(19) Europäisches Patentamt
European Patent Office
Office européen des brevets

(11) Publication number: **0 150 227**
**A1**

(12) # EUROPEAN PATENT APPLICATION

(21) Application number: **83307948.6**

(22) Date of filing: **23.12.83**

(51) Int. Cl.⁴: **C 07 K 5/06,** C 12 Q 1/36

(43) Date of publication of application: **07.08.85**
**Bulletin 85/32**

(84) Designated Contracting States: **CH DE FR GB IT LI NL**

(71) Applicant: **FUJIREBIO KABUSHIKI KAISHA** ***also trading*** **as FUJIREBIO INC., 6-7, Shimoochiai 4-chome Shinjuku-ku, Tokyo 161 (JP)**

(72) Inventor: **Sugiyama, Masami, 9-19, Motoyokoamamachi 2-chome, Hachioji-shi Tokyo (JP)**
Inventor: **Shibuya, Hiroko, SRL Akishimaryo 386-1, Miyazawacho, Akishima-shi Tokyo (JP)**
Inventor: **Kasahara, Yasushi, 310, 4-4, Nagayama 3-chome, Tama-shi Tokyo (JP)**

(74) Representative: **Silverman, Warren et al, HASELTINE LAKE & CO. Hazlitt House 28 Southampton Buildings Chancery Lane, London WC2A 1AT (GB)**

(54) **Dipeptide derivatives and their use in determining the activity of carboxypeptidase A.**

(57) Dipeptide derivatives useful as substrates for colourimetrically or spectrophotometrically measuring the activity of carboxypeptidase A have the following general formula

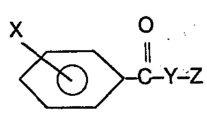

wherein X is OH in the ortho or para position, or $NH_2$ or $CH_3O$ in the para position, Y is

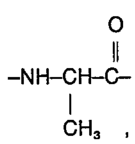 , 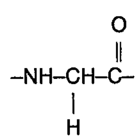 , 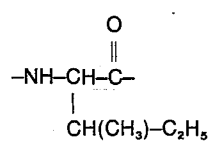 or

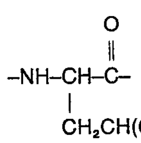 and Z is 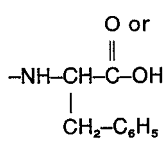 or 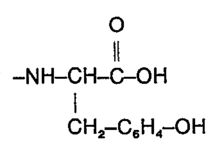

EP 0 150 227 A1

# "DIPEPTIDE DERIVATIVES AND THEIR USE IN DETERMINING THE ACTIVITY OF CARBOXYPEPTIDASE A"

This invention relates to novel dipeptide derivatives and to their use as substrates for measuring the activity of carboxypeptidase A (hereinafter referred to as CP-A for short).

CP-A is a protein-decomposing enzyme which is found in the pancreas and in blood serum. The activity of CP-A depends on the disease which is present and its extent. Accordingly, by measuring the activity of the CP-A, the extent ot which a disease has spread can be measured.

Our European Patent Application EP-A1-0083857 discloses two derivatives of hippuryl-L-phenylalanine having the following general formula

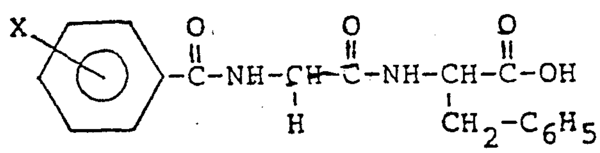

wherein X is OH or $CH_3O$. These dipeptides are useful as substrates for measuring the activity of carboxypeptidase A. The activity measuring method which has been described comprises the steps of:

(1) bringing into contact with each other in a liquid medium containing CP-A, (i) such a hippuryl-L-phenylalanine derivative as substrate, (ii) hippuricase, (iii) 4-amino-antipyrine and (iv) an oxidising agent capable as such of oxidising X-benzoic acid produced *in situ* to yield a substance which reacts with 4-aminoantipyrine present to yield a quinonimine dye;

(2) colourimetrically measuring the concentration of the quinonimine dye and

(3) calculating the activity of said enzyme from the concentration of the quinonimine dye.

It is an object of this invention to provide

alternative substrates and/or alternative activity measuring procedures for determining the activity of carboxypeptidase-A, which is/are able to offer like or superior effectiveness in the determination of the activity of carboxypeptidase-A.

According to one aspect of this invention, there are provided dipeptide derivatives which possess the following general formula:

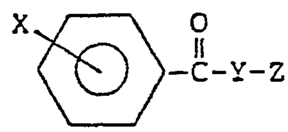 (I)

wherein X is OH in the ortho or para position, or $NH_2$ or $CH_3O$ in the para position, Y is

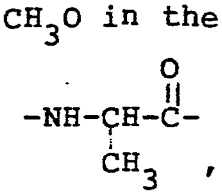 , 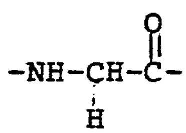 , 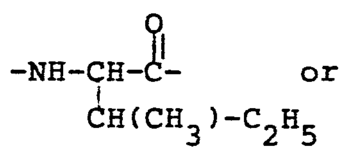 or

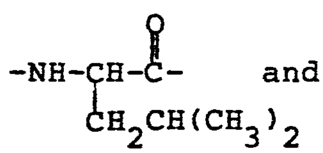 and Z is 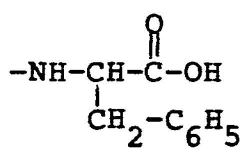 or 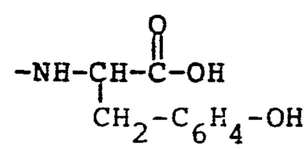 .

with the proviso that when X is OH or $CH_3O$, Y is not

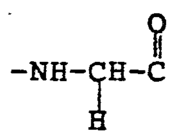 with Z being 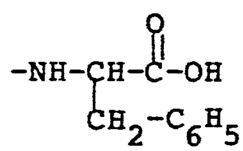

Specific examples of dipeptide derivatives embodying this invention are the following compounds:

1.

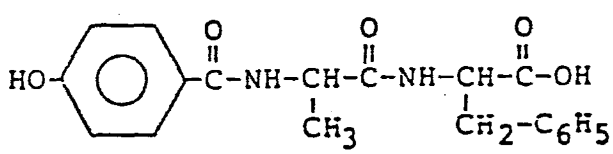

(p-hydroxybenzoyl-L-alanyl-L-phenylalanine)

2.

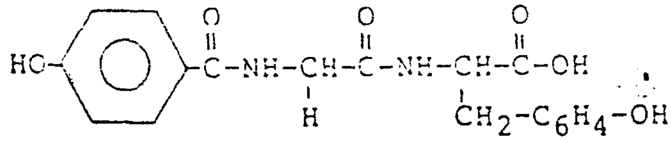

(p-hydroxybenzoylglycyl-L-tyrosine)

3.

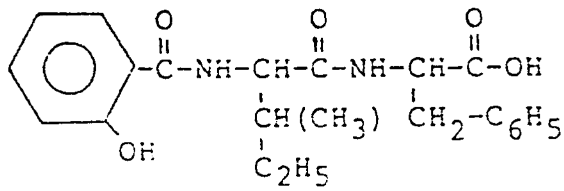

(o-hydroxybenzoyl-L-isoleucyl-L-phenylalanine)

4.

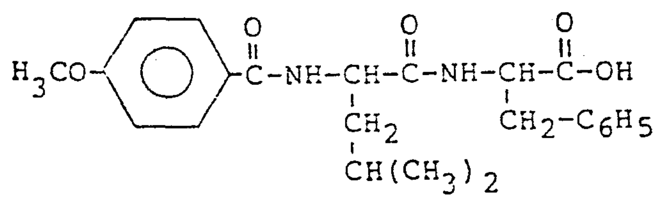

(p-methoxybenzoyl-L-leucyl-L-phenylalanine)

5.

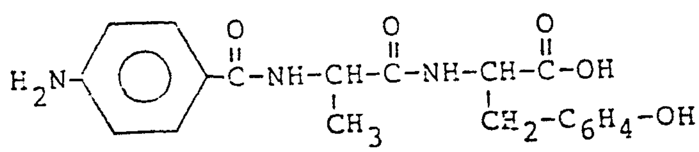

(p-aminobenzoyl-L-alanyl-L-tyrosine)

According to a second aspect of this invention there are provided methods for determining colourimetrically or spectrophotometrically the activity of CP-A. Thus a first method comprises the steps of

(1) bringing into contact with each other in a liquid medium containing the enzyme carboxypeptidase A, (i), as substrate, a dipeptide derivative of the invention, (ii) hippuricase, (iii) 4-aminoantipyrine and (iv) an oxidising agent capable as such of oxidising X-benzoic acid, obtainable from the dipeptide produced _in situ_ to yield a substance which reacts with 4-aminoantipyrine present to yield a quninonimine dye;

(2) colourimetrically measuring the concentration

of the quinonimine dye and

(3) calculating the activity of said enzyme from the concentration of the quinonimine dye.

A second method comprises the steps of

(1) bringing into contact with each other in a liquid medium containing the enzyme carboxypeptidase A, (i), as substrate, a dipeptide derivative of the invention (ii) hippuricase, (iii) NADPH or NADH and (iv) a benzoate hydroxylase having an identity corresponding to the meaning and position of X in the formula of the dipeptide derivative;

(2) spectrophotometrically measuring the decrease in Absorbance by NADPH or NADH at about 340 nm of light;

(3) calculating the activity of said enzyme from the decrease in absorbance by NADPH or NADH.

By the reference to the benzoate hydroxylase having an identity corresponding to the meaning and position of X in the formula of the dipeptide derivative, it is meant for example, that if X is p-OH, then a p-hydroxybenzoate hydroxylase will be used.

A third method comprises the steps of:

(1) bringing into contact with each other in a liquid medium containing the enzyme carboxypeptidase A, (i), as substrate, a dipeptide derivative of the invention wherein X is essentially $-NH_2$ and (ii) hippuricase;

(2) adding $NaNO_2$ to the reaction mixture to produce a diazonium compound;

(3) adding a coupling agent to produce an azo dye;

(4) colourimetrically measuring the concentration of the azo dye; and

(5) calculating the activity of said enzyme from the concentration of the azo dye.

In a third aspect, the invention provides a diagnostic composition for use in determining the activity of carboxypeptidase A, which, in its simplest

form, comprises a dipeptide compound of the invention and hippuricase.

When the composition is to be used in the first diagnostic method as aforesaid, it may additionally contain 4-aminoantipyrine and an oxidising agent capable as such of oxidising X-benzoic acid, <u>in situ</u> in the composition to yield a substance which reacts with 4-aminoantipyrine to yield a quinonimine dye. When the composition is to be used in the second diagnostic method it may additionally contain NADPH or NADH and a benzoate hydroxylase having an identity corresponding to the meaning and position of X in the dipeptide compound present.

The three diagnostic methods will now be described more fully with reference to specific reaction schemes. In the following, "Ala", "Phe", "Gly" and "Tyr" are used as abrreviations for "alanyl or alanine", "phenylalanyl or phenylalanine", "glycyl or glycine" and "tyrosyl or tyrosine", respectively, and further "NADH" means "reduced nicotinamide adenine dinucleotide" and "NADPH" means "reduced nicotinamide adenine dinucleotide phosphate".

1.

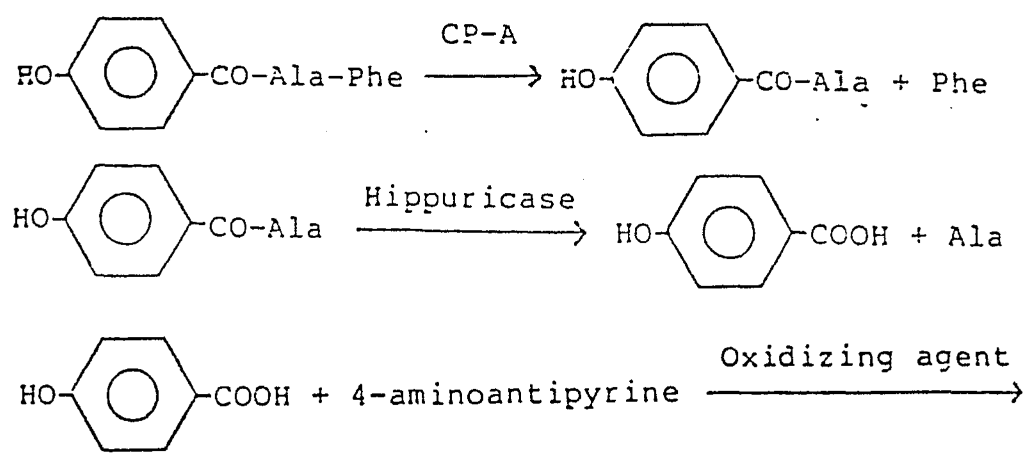


Quinonimine dye

The concentration of the quinonimine dye produced

may be colourimetrically measured, and the activity of CP-A is then calculated. As in EP1-A-0083857, the oxidising agent used may be, for example, periodic acid or a periodate perchloric acid or a perchlorate, potassium ferricyanide, ammonium ceric nitrate, chloramine T, chloramine B or chromium trioxide. Preferred procedures using the colourimetric method will be illustrated in the following Examples 6 and 7.

2.

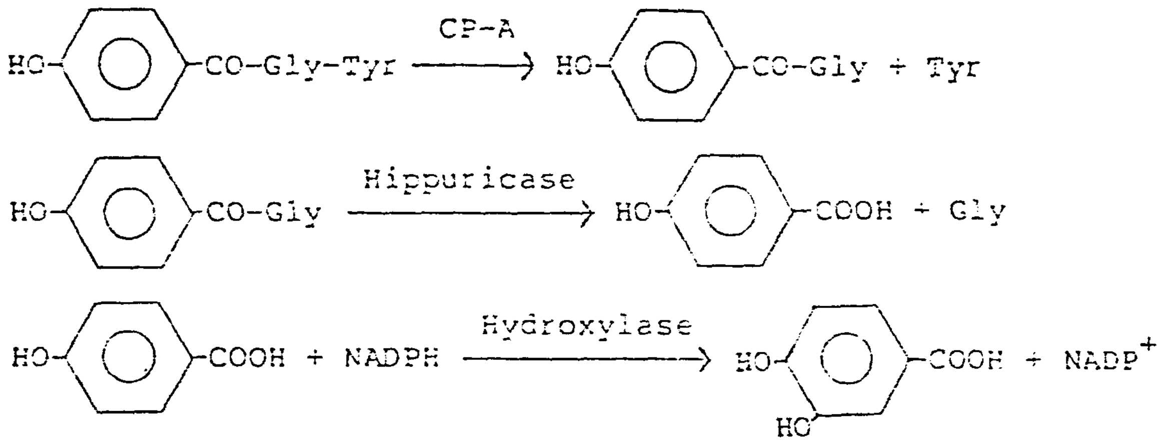


The decrease of the absorvbance by NADPH is spectrophotometrically measured, at an appropriate absorption band, preferably at 340 nm of light, during the course of the reaction using a reaction rate analyser, and the activity of CP-A is calculated. Preferred procedures using this spectrophotometric technique will be illustrated in the following Examples 8 and 9. In Example 10, NADH is used instead of NADPH in an otherwise like procedure.

3.

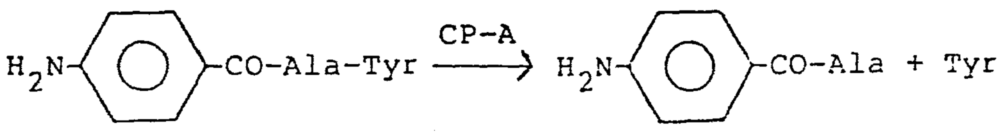

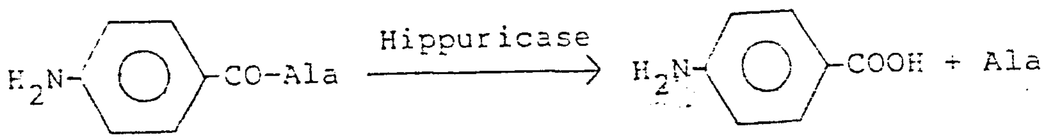


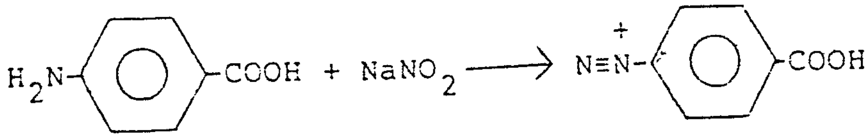

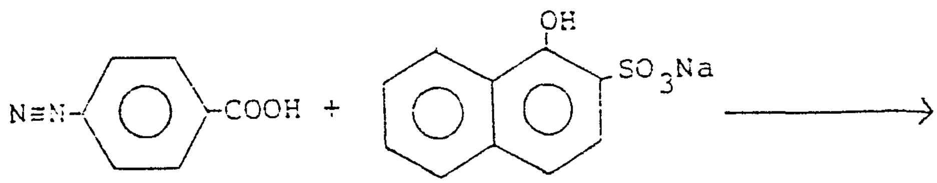

(Coupling reagent)

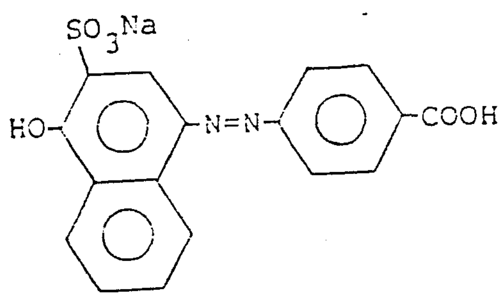

(Azo dye)

The concentration of the azo dye produced is colourimetrically measured, and the activity of CP-A is calculated. A preferred procedure using this colourimetric method will be illustrated in the following Example 11.

The dipeptide derivatives of this invention can be prepared by the following reaction:

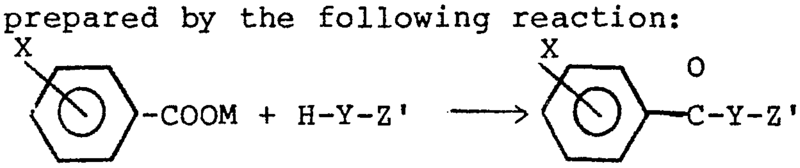 (II)

wherein X and Y have the aforesaid meanings wherein Z' is an esterification product of Z. This reaction is usually carried out in an organic solvent such as dicyclohexylcarbodiimide, and the compound of general formula (I) can be obtained by hydrolysis of the compound

having the general formula (II).

The following Examples illustrate this invention, Examples 1 to 5, illustrating the preparation of dipeptide derivatives of the present invention and the remaining Examples illustrating the use of such compounds in the determination of CP-A.

EXAMPLE 1

Synthesis of p-hydroxybenzoyl-L-alanyl-L-phenylalanine:

0.1 g (0.1 mol) of triethylamine was added to a suspension of 23.0 g (0.1 mol) of L-phenylalanine ethyl ester.HCl in 500 ml of dichloromethane. This mixture was placed in an ice bath and 22.3 g (0.1 mol) of carbobenzoxy-L-alanine, and then 19.2 g (0.1 mol) of 1-ethyl-3(3-dimethylaminopropyl) carbodiimide.HCl were added thereto. The mixture obtained was stirred for three hours and then were washed three times with a 0.1% aqueous solution of $Na_2CO_3$, three times with a 0.1 N HCl solution and then three times with distilled water. Thereafter, the mixture was dried over anhydrous sodium sulphate, and dichloromethane was removed from the mixture under reduced pressure to leave carbobenzoxy-L-alanyl-L-phenylalanine ethyl ester. This ester was dissolved in 260 ml of 25% HBr in glacial acetic acid, and the solution obtained was stirred for one hour. 5 Litre of absolute ethyl ether were added to this solution to produce a precipitate. The precipitate was filtered and washed with diethyl ether. L-alanyl-L-phenylalanine ethyl ester.HBr was thereby obtained.

17.3 g (0.05 mol) of L-alanyl-L-phenylalanine ethyl ester.HBr were suspended in 250 ml of dichloromethane, and to this suspension were added 5.1 g (0.05 mol) of triethylamine and 9 g (0.05 mol) of p-acetoxybenzoic acid. The mixture obtained was placed in an ice-bath and then 9.6 g (0.05 mol) of 1-ethyl-3(3-dimethylaminopropyl)carbodiimide HCl were added. The

mixture then obtained was stirred for three hours, and then was washed three times with a 0.1% aqueous solution of $Na_2CO_3$, three times with a 0.1 N HCl solution and three times with distilled water, before being dried over anhydrous sodium sulphate. Dichloromethane was removed from the dried mixture under reduced pressure to yield p-acetoxybenzoyl-L-alanyl-L-phenylalanine ethyl ester.

8.5 g (0.02 mol) of the ester thus obtained were dissolved in 50 ml of methanol, and to this solution, placed in an ice bath, were added 70 ml of a 1 N NaOH aqueous solution. The solution obtained was stirred for two hours at room temperature. After the pH of the solution had been adjusted to 7.0 with a 1 N HCl solution, the solvent was distilled off from the solution under reduced pressure. The residue of p-hydroxybenzoyl-L-alanyl-L-phenylalanine obtained was purified by absorption chromatography. 6.4 g of residue m.p. 211°C-211.6°C (decomposed) were obtained. Its infrared absorption spectrum is shown in Figure 1 of the accompanying drawings.

EXAMPLE 2

Synthesis of p-hydroxybenzoylglycyl-L-tyrosine:

21 g (0.1 mol) of carbobenzoxyglycine were dissolved in 500 ml of dichloromethane, and 24.5 g (0.01 mol) of L-tyrosine ethyl ester were added to this solution. The mixture obtained was placed on an ice-bath and 19.2 g (0.1 mol) of 1-ethyl-3(3-dimethylaminopropyl)carbodiimide.HCl were added. The reaction solution obtained was stirred overnight.

The reaction mixture was washed three times with a 0.1% aqueous solution of $Na_2CO_3$ and three times with distilled water, and then was dried over anhydrous sodium sulphate. Dichloromethane was removed from the reaction mixture under reduced pressure to leave behind carbobenzoxyglycyl-L-tyrosine ethyl ester.

250 ml of 25% HBr in glacial acetic acid were added to carbobenzoxyglycyl-L-tyrosine ethyl ester

obtained above, and, after the mixture obtained had been stirred for 30 minutes at room temperature, 5 litres of absolute ethyl ether were added to the mixture to produce a precipitate. The precipitate of glycyl-L-tyrosine ethyl ester.HBr obtained was washed with diethyl ether.

17.4 g (0.05 mol) of glycyl-L-tyrosine ethyl ester.HBr, 9 g (0.05 mol) of p-acetoxybenzoic acid and 5.1 g (0.05 mol) of triethylamine were dissolved in 250 ml of dichloromethane, and after the solution had been cooled to $0^{o}C$, 9.6 g (0.05 mol) of 1-ethyl-3(3-dimethylamino-propyl)carbodiimide.HCl were added to the solution, and the solution was stirred overnight. The reaction mixture obtained was washed with a 0.1% aqueous solution of $Na_2CO_3$ and with a 0.1 N HCl solution, and was dried over anhydrous sodium sulphate. Dichloromethane was removed from the mixture under reduced pressure and p-acetoxybenzoylglycyl-L-tyrosine ethyl ester was recovered.

8.6 g (0.02 mol) of p-acetoxybenzoylglycyl-L-tyrosine ethyl ester obtained above were dissolved in 50 ml of methanol, and to this solution were added 70 ml of a 1 N NaOH aqueous solution. The solution obtained was stirred for one hour at room temperature. The pH of the solution was adjusted to 7.0 with a 1 N HCl solution, and the solvent was distilled off from the solution under reduced pressure. The residue was dissolved in methanol, and the insoluble matter was filtered off. To the filtrate was added diethyl ether to produce a precipitate. This precipitate was filtered off and p-hydroxybenzoylglycyl-L-tyrosine in an amount of 5 g. m.p. $184^{o}C-210^{o}C$ (decomposed) was obtained. Its infrared absorption spectrum is shown in Figure 2 of the accompanying drawings.

EXAMPLE 3

Synthesis of o-hydroxybenzoyl-L-isoleucyl-L-phenylalanine:

4.6 g of o-hydroxybenzoyl-L-isoleucyl-L-phenylalanine were obtained by repeating a procedure analogous to that set out in Example 2, using 26.5 g of carbobenzoxyisoleucine instead of 21 g of carbobenzoxyglycine, 20 g of L-phenylalanine ethyl ester.HCl instead of 24.5 g of L-tyrosine ethyl ester.HCl, and 9 g of o-acetoxybenzoic acid instead of 9 g of p-acetoxybenzoic acid.

EXAMPLE 4

Synthesis of p-methoxybenzoyl-L-leucyl-L-phenylalanine:

The procedure of Example 2 was repeated, but using 26.5 g of carbobenzoxy-L-leucine instead of 21 g of carbobenzoxyglycine and 7.6 g of p-methoxybenzoic acid instead of 9 g of p-acetoxybenzoic acid, to obtain 3.6 g of p-methoxybenzoyl-L-leucyl-L-phenylalanine.

EXAMPLE 5

Synthesis of p-aminobenzoyl-L-alanyl-L-tyrosine:

4.4 g of p-aminobenzoyl-L-alanyl-L-tyrosine were obtained by repeating the procedure of Example 2, but using 22.3 g of carbobenzoxy-L-alanine instead of 21 g of carbobenzoxyglycine and 8.9 g of p-acetamidobenzoic acid instead of 9 g of p-acetoxybenzoic acid.

EXAMPLE 6

0.1 ml of serum containing carboxypeptidase A was added to 0.5 ml of a test reagent solution of pH 7.8 containing 10 mM p-hydroxybenzoyl-L-alanyl-L-phenylalanine, 0.1 M sodium chloride, 3000 U/l hippuricase, 2.5 mM 4-aminoantipyrine and 100 mM N-2-hydroxyethyl piperazine-N-2-ethane sulphonic acid. The mixture was incubated at 37°C for 20 minutes. 1.5 ml of a reaction termination solution containing 6.5 mM sodium periodate was added to this reaction mixture. The mixture obtained was incubated at 37°C for five minutes, so that quinonimine dye was formed. The absorbance of the reaction mixture was measured at a light wavelength of 505 nm. This absorbance is referred to as Absorbance A.

Separately, for a blank (comparison) test, in the procedure as mentioned above, 1.5 ml of a reaction termination solution containing 6.5 mM sodium periodide was added to the test reagent solution before 0.1 ml of serum containing carboxypeptidase A was added to the test reagent solution. The absorbance of the blank test reagent was then measured in the same manner as described above, at a light wavelength of 505 nm. This absorbance is referred to as Absorbance B.

The activity value (mU/ml) of carboxypeptidase A can be calculated by the following equation:

$$mU/m\ell = \frac{A - B}{\text{Molecular extinction coefficient } (\varepsilon)} \times \frac{1}{\text{Reaction time (5 min.)}} \times \frac{1}{\text{Light path length (cm)}} \times \frac{2.1}{0.1} \times 10^6$$

where $\varepsilon$ = 12000 (Molecular extinction coefficient of quinonimine dye)

By repeating the above-described test 20 times, using the same samples, the following results were obtained:

Activity value (mU/ml) = 5.1 (nmol/ml/min)

Coefficient of variation (CV) = 3.4%.

## EXAMPLE 7

The activity of CP-A was determined by repeating the procedure of Example 6, except that (a) p-hydroxybenzoylglycyl-L-tyrosine or (b) o-hydroxybenzoyl-L-isoleucyl-L-phenylalanine was used instead of p-hydroxybenzoyl-L-alanyl-L-phenylalanine.

The following results were obtained:

(a) Activity value (mU/ml) = 2.4 (nmol/ml/min)

(b) " = 1.2 "

(a) Coefficient of variation (CV) = 4.5%

(b) " = 6.0%.

EXAMPLE 8

0.05 mol of serum (the same as used in Example 6) was added to 0.5 ml of a test reagent solution (pH 8.0) containing 10 mM p-hydroxybenzoylglycyl-L-tyrosine, 3000 U/l hippuricase, 0.2 mM NADPH, 10000 U/l p-hydroxybenzoate hydroxylase and 100 mM boric acid.

The decrease in the absorbance by the solution was measured at 340 nm of light at a temperature of 37°C during the course of the reaction. There was used for the measurement a reaction rate analyser in which the reaction time was five minutes. The activity value (mU/ml) of CP-A was automatically printed out in the analyser.

The calculation of activity was performed according to the following formula:

$$mU/m\ell = \frac{\text{Decrease in absorbance at 340 nm}}{\text{Reaction time (5 min)}} \times \frac{1}{\text{Molecular extinction coefficient } (\varepsilon)} \times \frac{1}{\text{Light-path length (cm)}} \times \frac{0.55}{0.05} \times 10^6$$

wherein $\varepsilon = 6.22 \times 10^3$ (Molecular extinction coefficient of NADPH)

The results of the measurements, repeated 20 times, were as follows:

Activity value (mU/ml) = 6.2 (nmol/ml/min)

Coefficient of variation (CV) = 5.2%.

EXAMPLE 9

The activity of CP-A was determined by repeating the procedure shown in Example 8 except that o-hydroxybenzoyl-L-isoleucyl-L-phenylalanine was used instead of p-hydroxybenzoylglycyl-L-tyrosine, salicylate hydroxylase was used instead of p-hydroxybenzoate hydroxylase, and nicotinamide adenine dinucleotide in reduced form (NADH) was used instead of NADPH.

The following results were obtained:

Activity value (mU/ml) = 1.1 (nmol/ml/min)

Coefficient of variation (CV) = 7.0%

EXAMPLE 10

The activity of CP-A was determined by repeating the procedure shown in Example 8 with a mixture prepared by adding 0.05 ml of serum (the same as used in Example 6) to 0.5 ml of a test reagent solution (pH 8.0) containing 30 mM p-methoxybenzoyl-L-leucyl-L-phenylalanine, 1000 U/l hippuricase, 0.2 mM NADH, 20000 U/l p-methoxybenzoate hydroxylase and 100 mM phosphoric acid.

The following results were obtained:

Activity value (mU/ml) = 2.5 (nmol/ml/min)

Coefficient of variation (CV) = 4.7%.

EXAMPLE 11

0.05 ml of serum (the same as used in Example 6) containing CP-A was added to 0.5 ml of a test reagent solution (pH 8.0) containing 20 mM p-aminobenzoyl-L-alanyl-L-tyrosine, 0.5 M NaCl, 3000 U/l hippuricase and 100 mM boric acid. After this mixture had been allowed to stand at 37°C for 20 minutes, 0.5 ml of a solution containing 2 mM $NaNO_2$ and 1 mM NaOH was added to the mixture, and the mixture was allowed to stand at room temperature for 10 minutes. 0.5 ml of a reagent solution containing 1 mM potassium 1-naphthol-2-sulphate, 50 mM boric acid and 200 mM NaOH was added to the mixture. The absorbance by the solution was measured at 505 nm of light. This absorbance is referred to as Absorbance A.

Absorbance B was measured by repeating the procedure as shown above but using 0.05 ml of an aqueous solution containing 10 uM p-aminobenzoic acid instead of 0.05 ml of serum, and Absorbance C was measured by repeating the procedure as shown above but using 0.05 ml of distilled water instead of 0.05 ml of serum.

The activity value (mU/ml) of CP-A can be

calculated by the following equation:

$$mU/m\ell = \frac{A - C}{B - C} \times 10$$

By repeating the above-described test 20 times, the following results were obtained:

Activity value (mU/ml) = 1.7 (nmol/ml/min)

Coefficient of variation (CV) = 5.3%.

Claims:

1. A dipeptide derivative having the following general formula:

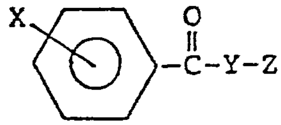

wherein X is OH in the ortho or para position, or $NH_2$ or $CH_3O$ in para position, Y is

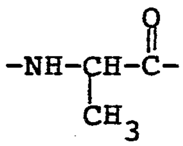 , 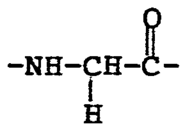 , 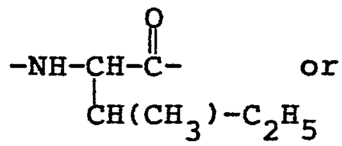 or

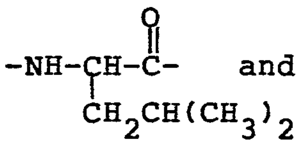 and Z is 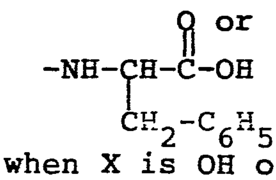 or 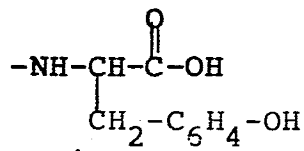

with the proviso that when X is OH or $CH_3O$, Y is not

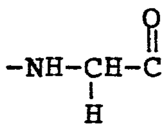 with Z being 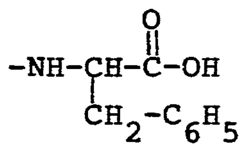

2. p-hydroxybenzoyl-L-alanyl-L-phenylalanine.

3. p-hydroxybenzoylglycl-L-tyrosine.

4. o-hydroxybenzoyl-L-isoleucyl-L-phenylalanine.

5. p-methoxybenzoyl-L-leucyl-L-phenylalanine.

6. p-aminobenzoyl-L-alanyl-L-tyrosine.

7. A method of determining the activity of carboxypeptidase A comprising the steps of

(1) bringing into contact with each other in a liquid medium containing the enzyme carboxypeptidase A, (i), as substrate, a dipeptide derivative as defined in claim 1, (ii) hippuricase, (iii) 4-aminoantipyrine and (iv) an oxidising agent capable as such of oxidising X-benzoic acid, obtainable from the dipeptide and produced *in situ* to yield a substance which reacts with 4-aminoantipyrine present to yield a quinonimine dye;

(2) colourimetrically measuring the concentration of the quinonimine dye and

(3) calculating the activity of said enzyme from the concentration of the quinonimine dye.

8. A method as claimed in claim 7, wherein said substrate is selected from p-hydroxybenzoyl-L-alanyl-L-phenylalanine, p-hydroxybenzoylglycyl-L-tyrosine and o-hydroxybenzoyl-L-isoleucyl-L-phenylalanine.

9. A method of determining the activity of carboxypeptidase A comprising the steps of:

(1) bringing into contact with each other in a liquid medium containing the enzyme carboxypeptidase A, (i), as substrate, a dipeptide derivative as defined in claim 1 (ii) hippuricase, (iii) NADPH or NADH and (ii) a benzoate hydroxylase having an identity corresponding to the meaning and position of X in the general formula of the dipeptide derivative;

(2) spectrophotometrically measuring the decrease in absorbance by NADPH or NADH at about 340 nm of light;

(3) calculating the activity of said enzyme from the decrease in absorbance by NADPH or NADH.

10. A method as claimed in claim 9, wherein the substrate (i) is p-hydroxybenzoylglycyl-L-tyrosine, o-hydroxybenzoyl-L-isoleucyl-L-phenylalanine or p-methoxybenzoyl-L-leucyl-L-phenylalanine and the benzoate hydroxylase (ii) is p-hydroxybenzoate hydroxylase, salicylate hydroxylase or p-methoxybenzoate hydroxylase respectively.
espectively.

11. A method of determining the activity of carboxypeptidase A comprising the steps of:

(1) bringing into contact with each other in a liquid medium containing the enzyme carboxypeptidase A, (i), as substrate, a dipeptide derivative as claimed in claim 1, wherein X is essentially $-NH_2$ and (ii) hippuricase;

(2) adding $NaNO_2$ to the reaction mixture to

produce a diazonium compound;

(3) adding a coupling agent to produce an azo dye;

(4) colourimetrically measuring the concentration of the azo dye; and

(5) calculating the activity of said enzyme from the concentration of the azo dye.

12. A method as claimed in claim 11, wherein the substrate (i) is p-aminobenzoyl-L-alanyl-L-tyrosine.

13. A diagnostic composition for use in determining tghe activity of carboxypeptidase A which comprises a dipeptide compound as defined in Claim 1 and hippuricase.

14. A diagnostic composition as claimed in claim 13, which additionally contains 4-aminoantipyrine and an oxidising agent capable as such of oxidising X-benzoic acid, wherein X has the meaning given in claim 1, in situ in the composition to yield a substance which reacts with 4-aminoantipyrine to yield a quinonimine dye.

15. A diagnostic composition as claimed in claim 13, which additionally contains NADPH or NADH and a benzoate hydroxylase having an identity corresponding to the meaning and position of X in the dipeptide compound of the composition.

0150227

1/2

# FIG. 1

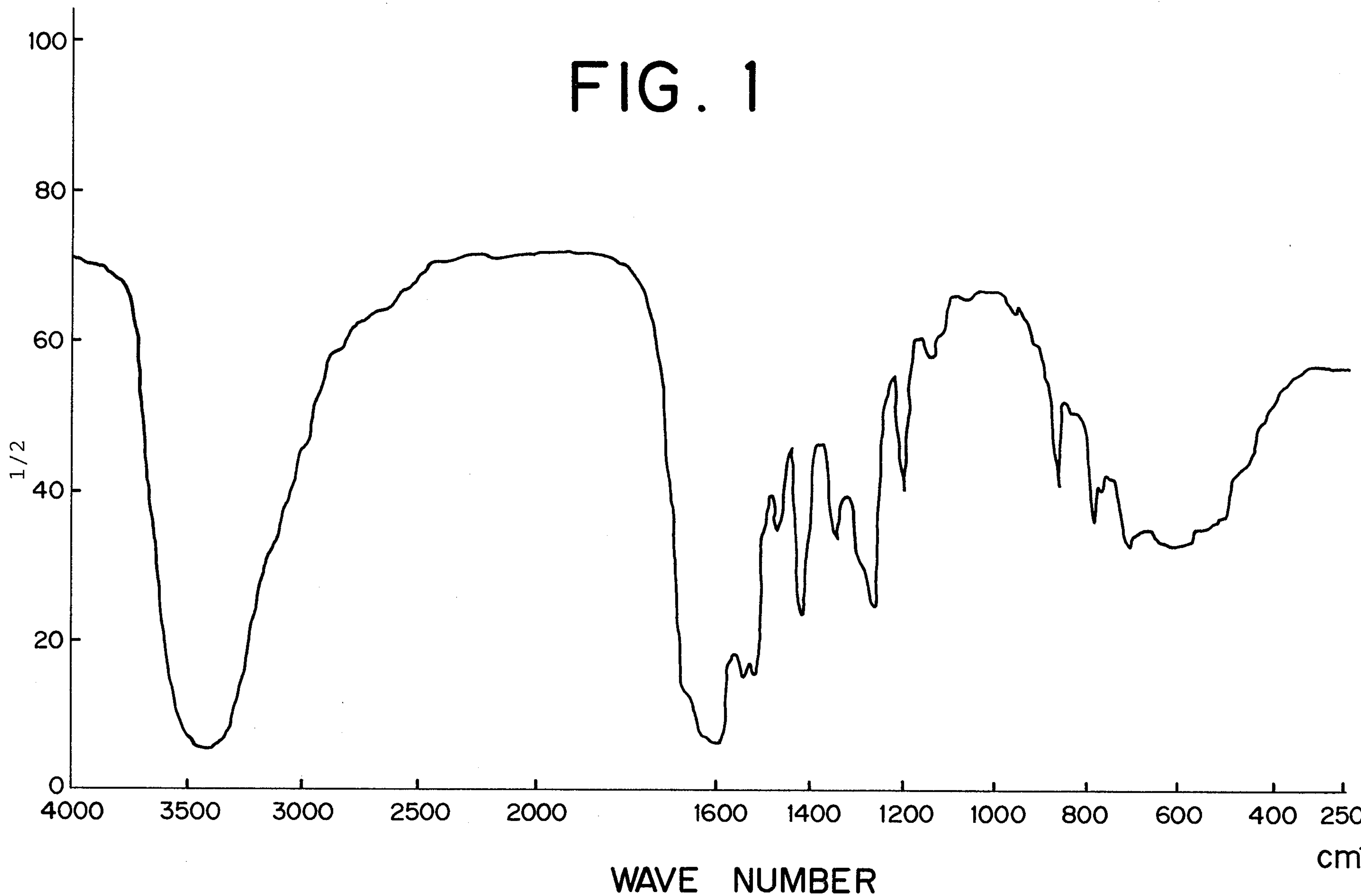


1/2

# FIG. 2

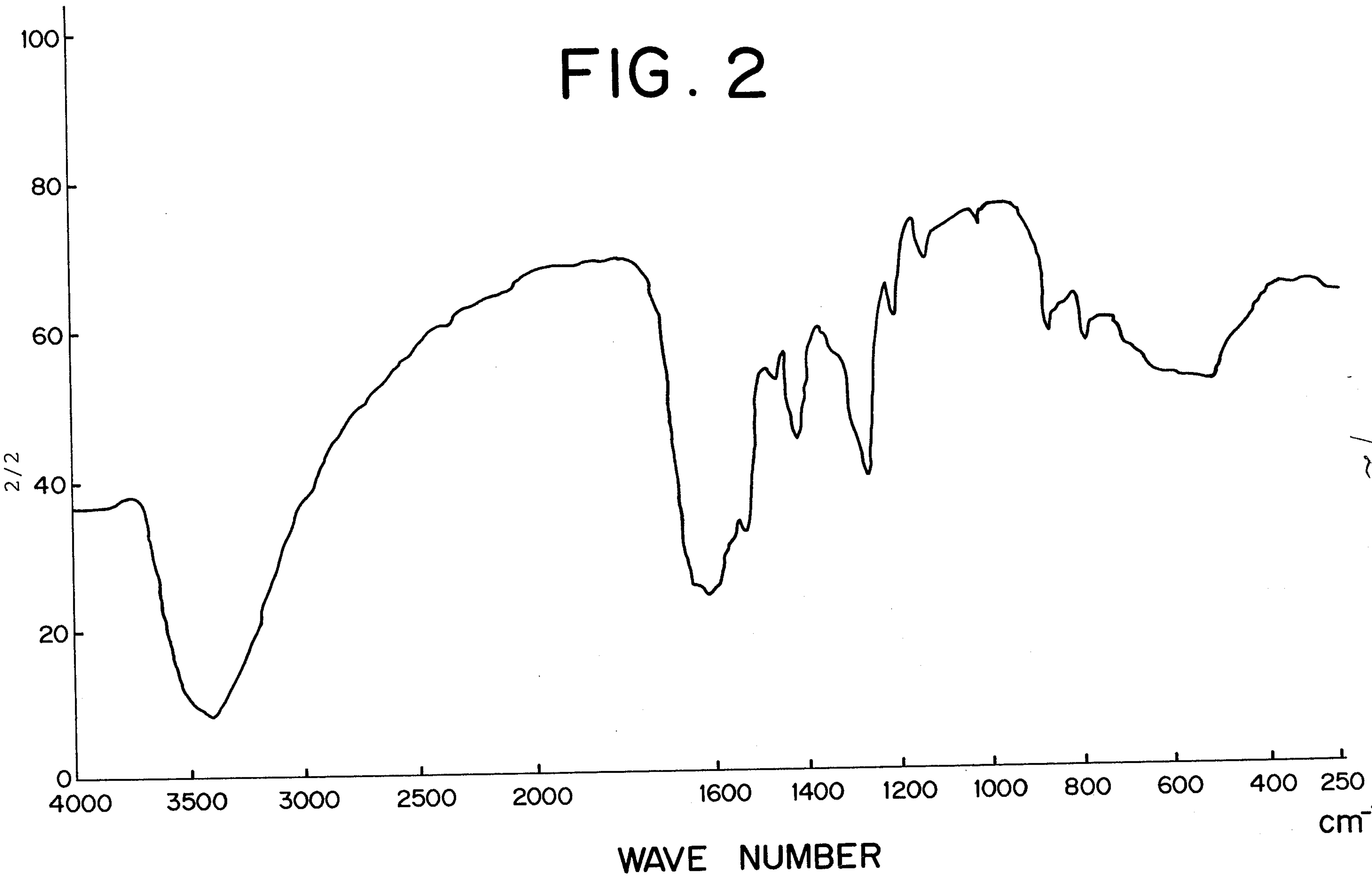


2/2



0150227

European Patent Office

# EUROPEAN SEARCH REPORT

0150227

Application number

EP 83 30 7948

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,Y | EP-A-0 083 857 (FUJIZOKI PHARM.) * Whole document * | 1-15 | C 07 C 103/52<br>C 12 Q 1/36 |
| Y | CHEMICAL ABSTRACTS, vol. 95, no. 7, 17th August 1981, page 766, no. 62664q, Columbus, Ohio, US; A.M. EL-NAGGAR et al.: "Synthesis of some 2-hydroxybenzoyl di- and tripeptide derivatives" & EGYPT. J. CHEM. 1979 (Pub. 1980). 22(5), 397-401 * Abstract * | 1-15 | |
| Y | ARCHIV DER PHARMAZIE, vol. 299, no. 2, February 1966, pages 179-188, Weinheim DE; K.-H. FRÖMMING et al.: "Die Darstellung einiger peptidartiger Derivate der Salicylsäure" * Whole article, particularly pag 181, example XXIV * | 1-15 | TECHNICAL FIELDS SEARCHED (Int. Cl. 3)<br>C 07 C 103/00 |

The present search report has been drawn up for all claims

| Place of search | Date of completion of the search | Examiner |
|---|---|---|
| THE HAGUE | 30-08-1984 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document